# EUROPEAN PATENT APPLICATION

(11) **EP 1 084 709 A1**
(43) Date of publication of application: **21.03.2001**
(21) Application number: 99203056.9
(22) Date of filing: 17.09.1999
(51) Int. Cl.: A61K 39/00, C12N 15/74

(54) **Oral recombinant lactobacilli vaccines**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: Shaw, David, Michael, HP3 OJX Bovingdon, Hertfordshire (GB); Leer, Robert Jan, 3904 PN Veenendaal (NL); Pouwels, Peter, 2289 AJ Rijswijk (NL)
(74) Representative: Wright, Simon Mark

(57) **Abstract**

The present invention relates to an oral vaccine comprising recombinant lactic acid bacteria expressing heterologous antigen in vivo intracellularly and/or the surface of the lactic acid bacterium as specific immunogenicity eliciting component for eliciting immunogenicity against the heterologous antigen, characterised in that the recombinant lactic acid bacterium is a *Lactobacillus plantarum*.

Preferably, the recombinant *Lactobacillus plantarum* comprises an expression vector capable of expressing the heterologous antigen intracellularly and/or such that the heterologous antigen is exposed on the cell surface under conditions present in the gastrointestinal tract.

The recombinant *Lactobacillus plantarum* is preferably a recombinant *Lactobacillus plantarum* 256.

The invention also relates to a recombinant *Lactobacillus plantarum*, more specifically a recombinant strain of *Lactobacillus plantarum* 256, for use in the vaccines of the invention; as well as to an expression vector suitable for intracellular expression or exposure of a heterologous antigen encoded thereon, said expression vector providing expression in a *Lactobacillus plantarum* of the heterologous antigen under conditions existing in the gastrointestinal tract.

## Description

### Summary of the invention

The subject invention lies in the field of vaccine development. Specifically the invention is concerned with the development of oral vaccines. The invention involves the use of recombinant non pathogenic bacteria as carriers of an antigen capable of eliciting immune reponse after oral application. More specifically the invention covers the use of a lactic acid bacterium of the genus *Lactobacillus.* The lactic acid bacterium concerned is classified as a *Lactobacillus plantarum.* The antigen is expressed intracellularly and/or exposed on the surface of the Lactobacillus. The vaccine is a live vaccine in that it uses live microorganisms as carrier of the antigen. The invention also covers new recombinant *Lactobacillus plantarum* and expression vectors for use therein.

### Background to the invention.

For some time the skilled person has been entertaining the idea of development of an oral vaccine based on a non pathogenic carrier rather than using the currently commercially applied attenuated pathogenic microorganisms such as *Salmonella typhimurium* and *Mycobacterium bovis.* The development of such attenuated strains is tedious and lengthy. It requires development of recombinant strains that are no risk to the environment or individual due to elimination of the pathogenicity. They must however remain immunogenically active to a sufficient degree for protective immune reponse to occur. Also a problem lies in the development of genetically stable recombinant strains. Also the use of an originally pathogenic microorganism carries the risk that immune reaction against the carrier itself will reduce the efficacy of a booster vaccine as will the presence of preexisting antibodies if the vaccinee has already been subjected to challenge by the carrier bacteria. Finally a large group of individuals will be more at risk using partially virulent carrier strains than using other carrier systems. This includes infants, the elderly and immune compromised individuals. In short there is a long felt need specifically for vaccines that are safe and effective.

Mucosal vaccines i.e. vaccines using the mucosal delivery route (vide the section "definitions" below) appear to offer several advantages over systemic inoculation. They can be used to generate IgA based immune response as a first line of defence. The protective immune response to mucosal infections is strongly dependent on the production of secretory IgA molecules. Such molecules are produced locally and are transported to the mucosal secretion products. A lot of effort is thus directed at vaccines capable of efficiently inducing protective local immune responses.

Another category of mucosal vaccines that could be extremely useful actually aims at systemic immune reponse induction via mucosal application of the vaccine rather than parenteral application. Ideally the immune response should be equal to that of parenteral vaccines to be sufficiently effective.

The systemic application of antigen however may not work, or may not work reliably, in stimulating the mucosal immune system. Unfortunately neither does oral administration of specific antigens result in sufficient immune response if it even results in any response at all. This is caused by poor adsorption, rapid degradation in the gastrointestinal tract of the antigen and tolerance induction to the host. So alternatives need to be addressed.

Among the options attempted is the production of protective carrier systems like iscoms, microspheres and liposomes and the use of harmless live microrganisms for local production (i.e. *in situ* in the body) of desired antigens in vivo.

The group of lactic acid bacteria has seemed a suitable starting point when looking for a group of harmless microorganisms to be used as carrier in view of the large scale use thereof already in the food production technology. They form a group of food grade (i.e. usually with GRAS-status) gram positive microorganisms suitable for human consumption. They have a long record of safe use in food products. An additional interesting aspect in their favour is of course the absence of LPS as opposed to the presence thereof in pathogenic microorganisms currently applied as carrier. Thus no problems with endotoxic shock risks are anticipated. So they are apparently safe to apply via the mucosal route. There is an abundance of information concerning large scale production thereof. So the upscaling of production with a view to commercial application should be feasible.

In addition, lactic acid bacteria are known to adhere to mucosal surfaces. They are also being produced as probiotics due to the colonising, i.e. capable of settling and or growing, potential of a number of strains of the cavities such as mouth, urogenital or gastrointestinal tracts, where they play a role in maintaining a balanced normal microflora. To date however no oral vaccine on the basis of non pathogenic bacteria is commercially available.

In the field of mucosal vaccines one must distinguish nasal and vaginal vaccines on the one hand and oral vaccines on the other. The environment the oral antigens are faced with is eminently more hostile than the nasal environment and to date it has been demonstrated that vaccines capable of eliciting immune reactions in nasal application have not often been successful upon oral application. Thus there is a large amount of trial and error involved in developing an oral vaccine.

Numerous nasal vaccines of non pathogenic carriers have been suggested in the prior art, but due to the non predictive character of efficacy thereof these disclosures cannot be considered relevant when faced with the problem of developing an oral vaccine.

For instance, the use of recombinant *Lactobacillus casei* and *Lactobacillus plantarum* strains as or in nasal vaccines have been described in the art, which teaches that both are equally suitable for this route of administration. Surprisingly, in the present invention, it has been found that (*inter alia*) certain *Lactobacillus plantarum* strains are suitable for use as or in an oral vaccine. In contrast - as further discussed below - the art has not been able to obtain comparable results with the *L .casei* strains that according to the art could be used successfully as nasal vaccine (Pouwels et al., J. Biotech. 1996 44:183-192, Pouwels et al., Int. J. Food Microbiol. 1998 41:155-167).

There are 3 categories of lactic acid bacteria based vaccines that have been suggested in the prior art as potentially being suitable. For two of these categories oral vaccines appear to be potentially feasible. Firstly there are the *Lactococcus lactis* based vaccines and secondly the *Streptococcus gordonii* based vaccines. Both categories have various advantages and disadvantages which are well documented in both the Pouwels and Mercenier review articles (Mercenier A. in Probiotics: A critical review, Horizon Scientific Press, Wymondham, U.K. 1999, pp 113-127; Pouwels et al., Int. J. Food Microbiol. 1998 41:155-167).

The third category is that of the *Lactobacillus* based vaccines. This category comprises illustrations of nasally introduced recombinant Lactobacilli capable of inducing immune response. This category however as yet does not illustrate any actual achievement of any orally introduced recombinant Lactobacilli actually exhibiting an immune response. Such disclosures are thus numbered amongst many speculative disclosures on the application of recombinant non pathogenic bacteria expressing heterologous antigen in vivo for producing a significant immune response (vide the section "definitions" below) to the heterologous antigen upon oral application of the bacterium.

Our study revealed a prior art disclosure of an unsuccesful experiment involving oral introduction into mice of a recombinant *Lactobacillus* expressing a heterologous antigen intracellularly. More specifically *Lactobacillus plantarum* 80 expressing *E*. *coli* beta-galactosidase intracellularly were introduced orally into mice on days 0,1 and 2. Subsequent oral boosting occurred after a 4 week interval. This experiment revealed no significant antibody responses (vide the section "definitions" below) to beta-galactosidase. Beta galactosidase was the heterologous antigen being expressed and this result was in contrast to intraperitoneal results where immune reaction was achieved, i.e. using the same bacterial strain, the same antigen and the same expression system.. This was reported in Wells et al., Antonie van Leeuwenhoek 1996 70:317-330. Numerous explanations for this failure can be put forward, however none of these have actually been tested to clarify the issue of lack of good results for oral vaccination and it is thus not at all clear whether the use of a *Lactobacillus* could result in immune protection even if the system were adapted vis a vis the disclosed *Lactobacillus plantarum* 80 system.

In addition to the above disclosure further disclosures directed at the potential use of recombinant *Lactobacillus plantarum* have been provided. We point out in this regard patent application WO99/11284 and a review article of Mercenier of 1999.

WO99/11284 suggests an oral vaccine can be produced using lactic acid bacteria. The embodiment suggested seems to be one where soluble protein is secreted from the recombinant microorganism. No mention of surface exposition or intracellular production is provided. A further aspect of relevance is that a large number of lactic acid bacteria are speculatively put forward as being useful. The suggested bacteria are *Lactobacillus delbruekii, Lactobacillus casei, Lactobacillus fermentum, Lactobacillus plantarum, Lactobacillus paraplantarum, Lactobacillus pentosus, Lactobacillus coryniformis, Lactobacillus brevis, Lactobacillus leichmannii* and strains of *Lactobacillus* isolated from intestinal flora such as *Lactobacillus rhamnosus* 901. The latter is indicated as being especially preferred as it possesses resistance both to acid and bile juices. Another lactic acid bacterium specifically mentioned in the examples is *Lactobacillus plantarum* 8826. No indication why such should be preferred is provided.

The lactic acid bacteria according to W099/11284 should produce urease as heterologous antigen. The urease would be capable of eliciting immune response. No other antigen is suggested. No illustration of *in vivo* successful induction of immune response is provided. The example supposedly illustrating such is merely speculative in nature and no actual results are provided. Also, according to this reference, the antigen (i.c. an urease) was excreted by the bacterial host into its surroundings as a "free", soluble protein ( i.e. not exposed on or otherwise associated with the surface of the bacterial host).

There is in fact serious reason to doubt the postulated method will be successful in providing an immune reaction. Numerous articles have been published pointing out that the secretion of soluble protein is unlikely to induce an immune response: therefore, this is not likely to be a suitable technique for providing a useful oral vaccine. It is for precisely this reason our efforts and those of others have recently been focussed on intracellular and surface exposed protein expression as source of heterologous antigen for eliciting immune response. This knowledge in combination with the absence of any experimental data in the patent application illustrates a lack of enablement of an oral vaccine using a *Lactobacillus* as host. At best it is a speculative disclosure of use of a *Lactobacillus* secreting soluble protein urease as heterologous antigen with a view to treating *Heliobacter pylori* associated disease. Nothing is disclosed concerning specific other antigens expected to exhibit specific problems e.g. problems related to the unpredictability of their surviving the aggressive environments of oral/gastrointestinal media.

Finally in the review article of Mercenier of 1999 it is described that numerous constructs expressing heterologous antigens i.a. TTFC have been introduced into *Lactobacillus plantarum* NIMB 8826. For all these recombinant Lactobacilli constructs nasal introduction in mice has resulted in immune response. In addition it is indicated oral tests have also been carried out. However the results of the oral experiments have not been presented. Thus it is not prior art whether any, some or all of the constructs de facto produced any significant immune reaction. Absence of data in combination with general knowledge concerning the extrapolation of data regarding nasal vaccines to oral vaccines together with previous failure using Lactobacilli as oral vaccines would appear to suggest lack of success. Certainly the general interpretation would be that there was not a reasonable expectation of success.

In support of the above conclusion we refer to the Pouwels et al 1998 disclosure. In this disclosure it is reported how expression vectors in *Lactobacillus casei* achieved good expression of heterologous antigen but merely provided fairly low levels of antibodies after oral introduction. Specifically in this respect it is mentioned this could be due to the poor viability of *L. casei.* Yet again however such parameter has not been further examined for actual relevance and numerous other aspects as such or in combination could be considered responsible for the failure of the recombinant *Lactobacillus casei* to provide protective immune response. Possibly the amount of expression *in vivo* was too low to actually elicit immune response or the antigen was not presented in a manner enabling it to induce an immune response.

In the experiments leading to the subject invention we in fact included the recombinant *Lactobacillus casei* to compare with the vaccine according to the invention and confirmed the aforementioned data concerning the lack of usefulness of a *Lactobacillus casei* based expression system as in vivo carrier of heterologous antigen. This finding also puts the disclosure of WO99/11284 in jeopardy as there it was suggested *Lactobacillus casei* could be an example of a useful host. Naturally this is extremely questionable on the basis of our findings.

To further support how extrapolation of data in this field is impossible we refer to previous experiments where non recombinant *L. plantarum* and non recombinant *L. casei* were compared for eliciting adjuvant activity. *L. casei* was shown to be the best microorganism eliciting in vivo adjuvant activity. Thus one could have speculated *L*. *casei* would be a preferred candidate for vaccine use over *L. plantarum* or any other strain of lactic acid bacteria. Numerous others were also tested and found not to elicit any adjuvant response whatsoever.

### Definitions.

In the present description, the terms and phrases mentioned below will have the following meaning, unless indicated otherwise:
- "*Bacterial host*" means the bacterium or bacterial strain that is used to express the desired antigen - e.g. via recombinant techniques - and/or that is administered to the body of a human or animal (mammal) in or as part of a vaccine according to the invention, in order to illicit an immune response against said antigen.

Depending on the specific context, *"bacterial host"* is used to designate the native strain transformed to express the antigen, the recombinant strain expressing the antigen (also referred to separately as *"recombinant host strain"*), or both.
- *"Mucosal delivery (route)"* of a vaccine means any route of administration to the body of a human or animal for which it is not required to puncture the skin ( e.g. as with intravenous, intramuscular, subcutaneously or intraperitoneal administration). Usually, this means that the vaccine is administered to the body via one of the body cavities, such that it comes into contact with the mucosa.

Herein, mucosal adminstration in particular refers to nasal, oral and/or vaginal administration.
- *"Mucosal vaccine"* means any vaccine suited, intended and/or formulated for mucosal delivery as defined above.
- *"Oral delivery (route)"* of a vaccine means any route of delivery to the body of a human or animal by which the vaccine can be presented to the gastrointestinal tract or any part thereof.

Usually, this will involve administration into or via the mouth into the g.i.tract. However, but for the purposes herein, *"oral administration"* also includes administration directly into the g.i. tract or into any part thereof, and in particular into the stomach, for instance using a tube or cathether.
- *"Oral vaccine"* means any vaccine suited, intended and/or formulated for oral delivery as defined above.
- A response (e.g. an antibody respone or immune response) is deemed *"significant"* when it leads to a detectable change or response in a human or animal, and in particular to a detectable immunological change or response, such as the production of antibodies, cytokines, lymphokines, etc..

Tests for determining whether a response is *"significant"* are known in the art of immunology and include, but are not limited to titration of antibody levels in biological samples using ELISA techniques, ELISPOT techniques and in vitro lymphocyte stimulation assays. Such techniques are usually carried out on a biological sample, such as a biological fluid or cell sample, obtained from the human or animal.

A *"significant"* response as used herein may be, but is not necessarily, also a *"protective"* response as defined below.
- A response (e.g. an immunological response against a pathogen or an antigen) is deemed *"protective"* when it is capable of protecting the human or animal which shows said response against said pathogen and/or against a pathogen associated with said antigen.
- An antigen is deemed *"exposed"* on a bacterial host ( also be referred to as *"(surface) exposition"* of the antigen) when it is present, forms part of, is attached to, and/or is otherwise associated with or detectable on (e.g. using a suitable immunological detection technique such as FACS or immunofluorescent microscopy) the surface of the bacterial host (e.g. the bacterial cell wall or envelope)

Most preferably, *"exposed"* also means that the bacterium - when presented to a cell of a human or animal that is capable of mediating an immune response (such as the cells of the g.i. tract mentioned below) during sufficient time and in a sufficient amount - is capable of illiciting a *"sufficient"* immune response against said antigen. For the remainder, all terms and phrases used herein will have their usual meaning in the art.

### Description of the invention.

In a first embodiment, the subject invention is directed at an oral vaccine comprising recombinant lactic acid bacteria expressing heterologous antigen *in vivo* intracellularly and/or exposed on the surface of the lactic acid bacterium as specific immunogenicity eliciting component for eliciting inmmunogenicity against the heterologous antigen, characterised in that the recombinant lactic acid bacterium is a *Lactobacillus plantarum,* and optionally at least one pharmaceutically acceptable carrier suitable for use in a formulation for oral delivery.
A *Lactobacillus plantarum* can readily be defined on the basis of known parameters for determination. Bergeys manual of determinative bacteriology and Vescovo et al, Ann. Microbiol. Enzimol. 43 261-284 1993 for example illustrates such. The skilled person can therefore readily determine whether a lactic acid bacterium is a *Lactobacillus plantarum.* Numerous *Lactobacillus plantarum* strains have been deposited at various institutes and are readily available.

The native strain selected as the bacterial host should preferably have GRAS status, and more preferably be food grade, although the invention is not limited thereto.

Also, the bacterial host used should allow - upon transformation with an approriate construct encoding an antigen, e.g. as described below - expression of the desired antigen either intracellularly and/or exposed on the surface as described herein.

Preferably the level of expression of the antigen - e.g intracellulary and/or exposed on the surface as determined by SDS-polyacrylamide gelelectrophoresis or FACS should be at least 1-5 % of total cell protein, or alternatively 80% or more, preferably 100% or more, of the level of expression provided by *L.plantarum* strain 256 under the same conditions and using the same expression vector.

Also, the bacterial host is preferably capable - upon oral administration, e.g. as part of or in a vaccine according to the invention - of settling in and/or colonizing at least part of the gastrointestinal tract, such as the mouth, the throat, the larynx, the gut, the small intestine, the large intestine, the ileum and/or the colon, or a combination thereof. According to one preferred embodiment, the bacterial host is such that it mainly settles in the intestine, more preferably in the small intestine, although the invention is not limited thereto.

In doing so, the recombinant *Lactobacillus plantarum* strain preferably exhibits a persistance in the individual to be immunized - upon oral administration and as determined by the presence of the strain in the faeces - exceeding 5 days, preferably exceeding 9 days, and suitably more than 15 or even 20 days, although the latter is not required, in particular when an administration regimen comprising the use of one or more booster immunisations as described below is envisaged.

A preferred embodiment exhibits a persistance longer than that of *L. plantarum* 80 and preferably longer than that of *L. plantarum* NCIMB 8826 under equivalent conditions. We can for example assess such objectively in the gastrointestinal tract of mice of the same strain, preferably of the same age under equivalent conditions of treatment. The options available are numerous and will be clear to a skilled person.

According to one aspect of the invention, the *L. plantarum* strain is not LMG 9211 (NCIMB 8826 as described by Mercinier above), DSM 4229 and/or *L. casei* 393.

According to another aspect of the invention, the *L.plantarum* strain is not NCIMB 8826 or *L. plantarum* 80.

According to a preferred embodiment, the bacterial host is chosen from the following *L. plantarum* strains: 256, LMG 1284, LMG 6907, LMG 8155, LMG 9205, LMG 9206, LMG 9208, LMG 9209, LMG 9210, LMG 9212, LMG 11405, LMG 11460, LMG 8095, LMG 8027, LMG 12167, LMG 13556, LMG 17552, LMG 18021, LMG 18023, LMG 18024, LMG 18027, LMG 18095, 386 (see Molin et al., 1993. J. Appl. Bacteriol. 74:314), 299 (see Molin et al., 1993. J. Appl. Bacteriol. 74:314), 105 (see Molin et al., 1993. J. Appl. Bacteriol. 74:314), 275 (see Molin et al., 1993. J. Appl. Bacteriol. 74:314), 299v (see WO 96/29083), So5 (see Johannson et al 1995. Vol 45:670), 36^{E} (see Johannson et al 1995. Vol 45:670), 79 (see Johansson et al 1995 Int. J. Food. Micro. 25:159), 107 (see Johansson et al 1995 Int. J. Food. Micro. 25:159), 98 (see Johansson et al 1995 Int. J. Food. Micro. 25:159), 95 (see Johannson et al 1995. Vol 45:670), 53 (see Johansson et al 1995 Int. J. Food. Micro. 25:159), 97 (see Johansson et al 1995 Int. J. Food. Micro. 25:159), 101 (see Johansson et al 1995 Int. J. Food. Micro. 25:159), 125 (see Johansson et al 1995 Int. J. Food. Micro. 25:159), 120 (see Johannson et al 1995. Vol 45:670), 44 (see Johannson et al 1995. Vol 45:670), (see Johannson et al 1995. Vol 45:670), CH, ATCC 8041, ATCC 10012, ATCC10776, WCFS, DF66 IIIa, DF66spez. -IVa, and/or the *L. plantarum* strains available from the Japanese Collection of Micro-organisms under accession numbers: 8341, 8342, 8343, 8344, 8345, 8346, 8347, 8348. These strains are either described in the art and/or are available from the pertinent culture collection under the accession number indicated.

Other suitable strains may be selected by the skilled person on the basis of one or more properties or factors such as:
- stability of the construct encoding the antigen in the bacterial host selected; level of expression of antigen in the bacterial host selected; regulation of expression of antigen in the bacterial host selected; site of expression of antigen in the bacterial host selected; stability of antigen produced; as well as
- the biochemical properties of the strain used, including but not limited to its sugar fermentation profile (API), cell wall composition, structure LTA, structure peptidoglycan, 16S RNA sequence, acid resistance, bile acid resistance, agglutination properties, adjuvanticity, immune modulating properties, in vitro adherence properties, mannose-specific adherence, presence of proteinaceous adherence factors, presence of mapA-like adherence factors, presence of large proteinaceous adherence factors with repeated amino acid sequences; and
- the interaction of the bacterial host with cells of the individual to which the host to be administered (i.e. as part of a vaccine according to the invention) including but not limited to its persistence (which is preferably as defined above), viability, in vivo expression of antigen and/or tissue-specific persistence.

In at least some, preferably most, and more preferably essentially all of these properties, the strain used should be essentially (at least) equivalent to the strains mentioned above, and more preferably to *L.plantarum* 256, i.e. as determined on the basis of tests/assays for these properties known per se in the art. After a suitable host has been selected, it may be transformed with a genetic construct as described herein, after which its suitability as an oral vaccine may be tested, i.e. using the tests and protocols described in the Experimental Part below. It is envisaged that on the basis of the description herein, and optionally after carrying out - for the purposes of confirmation- the tests described herein, the skilled person will be able to identify other *L. plantarum* strains suitable for use as or in vaccines of the invention.

Particularly preferred is *L. plantarum* strain 256.

A combination of one or more of the strains mentioned above may also be used.

A preferred embodiment of the invention covers a vaccine wherein the recombinant *Lactobacillus plantarum* comprises an expression vector capable of expressing the heterologous antigen intracellularly and/or such that the heterologous antigen is exposed on the cell surface under conditions present in the gastrointestinal tract.

Any embodiments of recombinant *Lactobacillus plantarum* in the form of a vaccine wherein the heterologous antigen is specific for inducing immunogenicity against a pathogenic microorganism are covered by the scope of the invention. Quite suitably such an embodiment expresses a heterologous antigen specific for mucosa colonising pathogens or pathogens entering the body via the mucosa, specifically via the oral route. More preferably any embodiment wherein the heterologous antigen is specific for a gastrointestinal tract colonising pathogen. Suitably as is illustrated in the Example a heterologous antigen specific for tetanus (*Clostridium tetanus*) is a particularly suitable candidate.

Naturally the invention only covers recombinant bacterial hosts (e.g. of *Lactobacillus plantarum*) comprising expression vectors capable of expressing the heterologous antigen intracellularly and/or such that the heterologous antigen is exposed on the cell surface to a degree sufficient to induce protective immunogenicity.

Preferably the vaccines are formulated such that a single dose is sufficient. However embodiments where multiple applications over a period of time, e.g. with a view to the persistance of the bacterial host in the g.i. tract, are also envisaged as falling within the scope of the invention. The provision of booster vaccinations is also envisaged as a potential embodiment with the vaccine formulations according to the invention.

A preferred administration regimen comprises one or more "initial" administrations in days 1 to 4, followed by one or more booster administrations in days 14 to 21, and optionally one or more further booster administrations in days 28 to 25. A single initial administration, followed by a single booster administration, within this time period, will generally be sufficient.

Surprisingly, in some cases, it has been found that - when a bacterial host is used with a persistance between 6 to 12 days - a significant immune response is obtained essentially only after the first booster administration. This was despite the fact that at the time of the first booster - i.e. days 14 to 21- the bacterial host (i.e. from the initial administration) was not longer detectable in the faeces of the individual. Reference is made to Figures 5a-5c, which show that identically primed mice were all boosted at the time the oral vaccine was re-administered, either 2,3 or 4 weeks after initial priming. In one aspect, the invention therefore relates to a method and preparations suited for such an administration and boosting regimen.

Any vaccine comprising a recombinant *Lactobacillus plantarum* comprising expression vectors capable of expressing the heterologous antigen intracellularly and/or such that the heterologous antigen is exposed on the cell surface to a degree exceeding that of the vector disclosed for *Lactobacillus plantarum* 80 beta galactosidase expression in *Lactobacillus plantarum* 80 is a preferred embodiment of the invention.

As high a degree of expression as possible without damaging the viability of the cell or the host to be vaccinated is envisaged. The higher the expression the less frequent the dosage is likely to be required for immunisation purposes. Naturally the dosage regime will not only depend on amount of antigen but also on antigen type and the presence or absence of other immunogenicity stimulating factors in the vaccine.

A high degree of expression can be achieved by using homologous expression and/or secretion signals on the expression vectors present in the recombinant *Lactobacillus plantarum* in the vaccine. Suitably expression regulating signals as present on the constructs in the examples are useful. Other expression signals will be apparent. The expression vectors can as will be obvious to a person skilled in the art be adjusted to optimise expression depending on the *Lactobacillus strain* it is incorporated in.

Surprisingly, we have found that expression vectors that gave reasonable amounts of expression in *Lactobacillus casei* but not to a degree sufficient for the recombinant *Lactobacillus casei* to be contemplated as an oral vaccine, were sufficient to provide expression to a degree sufficient for *Lactobacillus plantarum* to provide immunogenicity upon oral administration. Thus *Lactobacillus plantarum* comprising expression vectors capable of expression in *Lactobacillus casei* are covered. This was not expected to be the case. Firstly this could be considered to be the case due to the lack of result in *Lactobacillus casei* and secondly due to the unpredictability of degree of expression between various lactic acid bacteria.

A preferred embodiment of the invention is provided by a recombinant *Lactobacillus plantarum* when said Lactobacillus is a recombinant *Lactobacillus plantarum* 256. As is illustrated in the example such strain provides good results. As is also apparent from the examples *Lactobacillus casei* under equivalent conditions expressing the same antigen does not provide good results.

The antigen can be any antigen against which an immune response, more specifically a "significant" immune response and/or a "protective" immune response as defined above, is to be illicited in an animal, preferably a mammal, and/or a human. Although the invention is not limited thereto, the antigen will usually be associated with a pathogen, disease state and/or disorder of the human or animal to which the vaccine is to be administered.

Any antigen, antigenic component or epitope known per se that can be expressed in the microbial host - such that is either produced intracellularly and/or becomes exposed on the surface of the bacterium in accordance with the invention - can be used. Usually, this will be a peptide, a protein, or an antigenic part or fragment thereof, such as an epitope. As such, it may either be a native antigenic peptide or protein (or part, fragment or epitope thereof) or an antigenic analog or mutant thereof, for instance obtained synthetically or using recombinant DNA techniques.

For instance, according to the invention, recombinant bacteria and/or bacterial strains, as well as vaccines based thereon, may be provided that can be used to illicit - through oral administration - a significant immune response, and preferably a protective immune response, against antigens such as:
- human allergens;
- viral and/or bacterial antigens including - but not limited to - the gp160 envelope protein of the HIV virus, the surface glycoprotein of a *Leishmania* parasite, Shiga-like toxin, *Shigella* lipopolysaccharide antigen, *Escherichia coli* fimbrial antigen, a CFA antigen of an enterotoxigenic *Escherichia coli* strain, anthrax toxin, pertussis toxin, tetanus toxin; as well as
- antigens from such pathogens as herpes virus, rubella virus, influenza virus, mumps virus, measles virus, poliomyelitis virus, rotavirus, respiratory syncytial virus, *Campylobacter* species, *Chlamydial* organisms, species of the genus *Cryptosporidium,* cytomegalovirus, human immounodeficiency virus, *Actinomyces* species, *Entamoeba histolytica,* arenaviruses, arboviruses, *Clostridium botulinum,* species of the genus *Candida, Vibrio cholera, Cryptococcus neoformans,* EHEC strains of *E. coli* 0157:H7, 026:H11, 0111:H8 and 0104:H21, ETEC strains of *E*. *coli,* strains of *E.coli* shown to possess enteroinvaisiveness (EIEC), EPEC strains of *E.coli,* EAggEC strains of *E.coli.,* DAEC strains of *E.coli,* filoviridae, parvovirus, *Filarioidea, Staphylococcus aureus,* species of the genus *Clostridium perfringens, Helicobacter pylori,* Caliciviruses, *Giacardia lamblia, Neisseria gonorrhoeae,* hantaviruses, hepatitis viruses types A, B, C, D, E, *Legionellae* strains, *Mycobacterium leprae, Listeria monocytogenes,* species of the genus *Clostridium perfringens, Borrelia burgdorferi, Pseudomonas pseudomallei,* Epstein Barr virus, *Onchocerca volvulus,* Poxviruses, *Bordetella pertussis, Yersinia pestis, Coxiella burnetti,* rabies virus, *Treponema pallidium, Mycobacterium tuberculosis, Salmonella typhi,* eukaryotic parasite causing malaria, *pneumocystis pneumonia,* as well as agents causing toxoplasmosis.

Again, besides such native antigens and antigenic components (including antigenic parts, fragments or epitopes thereof), antigenic mutants or analogs thereof - obtained synthetically or via recombinant DNA techniques - may be used. Also, in a vaccin of the invention, a combination of two or more such antigens may be expressed, i.e. by a single type or strain of bacterial host, or by several different types or strains of bacterial host.

Of the above, antigens against and/or specific for rotavirus, respiratory synctial virus, Mycobacterium tuberculosis, human immunodeficeincy virus, E.coli, Vibrio cholera, streptococci and chlamydia are especially preferred for use as an antigen in the vaccines of the invention.

The antigen is preferably such that, upon expression, it still allows - at least to some extent, which may be reduced compared to the native strain - the recombinant bacterial host to settle in and/or colonize (part of) the gastrointestinal tract upon administration, and to persist there - when administered according to a regimen as described herein - for a time sufficient to provide a significant immune response against the antigen and/or the pathogen associated with it.

The antigen may be expressed in the bacterial strain using any expression system known per se that can provide for expression of the antigen in the recombinant bacterial host in a manner that makes the host suitable for use as or in a vaccine of the invention. This means that at least the antigen should be expressed intracellulary and/or such that it becomes exposed on the surface of the bacterial host, and at a suitable level, i.e. at least 1 - 5 % of total cell protein, as measured by SDS-polyacrylamide gel electrophoresis and standard protein staining.

Usually, the expression system will comprise a genetic construct comprising at least a nucleotide sequence encoding the desired antigen(ic component), operably connected to a promoter capable of directing expression of said sequence in the bacterial host. Suitably the antigen to be expressed can be encoded by a nucleic acid sequence adapted to preferred codon usage of the bacterial host as is common practice in the art of recombinant technology. The construct may further contain all other suitable elements of such constructs known per se, including enhancers, transcription initiation sequences, signal sequences, reporter genes, transcription termination sequences, etc., operable in the selected bacterial host.

The construct is preferably in a form suitable for transformation of the bacterial host and/or in a form that can be stably maintained in the bacterial host, such as a vector or plasmid. More preferably, a food grade construct is used.

A particularly preferred construct according to the invention comprises the multicopy expression vector described in PCT/NL95/00135, in which the nucleotide sequence encoding the antigen has been incorporated. As described in PCT/NL95/00135, such a construct is particularly suited for the expression of a desired protein or polypeptide in a lactic acid bacterium, in particular in a *Lactobacillus,* at a high level of expression, and also can be used advantageously to direct the expressed product to the surface of the bacterial cell.

More in particular, the constructs of PCT/NL95/00135 - as applied in the present invention- will be characterised in that the nucleic acid sequence encoding the antigen is preceded by a 5' non-translated nucleic acid sequence comprising at least the minimal sequence required for ribosome recognition and RNA stabilisation, followed by a translation initiation codon, said translation initiation codon being immediately followed by a fragment of at least 5 codons of the 5' terminal part of the translated nucleic acid sequence of a gene of a lactic acid bacterium or a structural or functional equivalent of said fragment, said fragment also being controlled by said promoter. The contents of PCT/NL95/00135, including the differing embodiments disclosed therein, are incorporated herein by reference.

Preferably, any alternative construct used in the invention provides a level of expression - e.g. intracellularly and/or exposed at the surface - that is at least comparable to the level provided by a vector of PCT/NL95/00135 in the same bacterial host under the same conditions.

The sequence encoding the antigen can be obtained from any natural source and/or can be prepared synthetically using well known DNA synthesis techniques. The sequence encoding the antigen can then (for instance) be incorporated in a suitable expression vector, such as the vectors described in PCT/NL95/00135, to provide a genetic construct of the invention, which is then used to transform the intended bacterial host strain, in a manner known per se, again for instance as described in PCT/NL95/00135.

The recombinant bacterial host thus obtained can then be cultured, upon which the harvested cells are used to formulate the vaccine, optionally after further purification and/or processing steps known per se, such as freeze-drying to form a powder.

The techniques used in providing the genetic constructs containing the antigen-encoding sequence and for transforming, culturing and harvesting the bacterial hosts are well known in the art, and are for instance described in PCT/NL95/00135 as well as in the standard handbooks, including Sambrook et al, "Molecular Cloning: A Laboratory Manual" (2nd ed.), Vols. 1-3, Cold Spring Harbor Laboratory (1989) of F.Ausubel et al, eds., "Current protocols in molecular biology", Green Publishing and Wiley Interscience, New York (1987).

The vaccine comprising the bacterial host can be formulated in a manner known per se, such as for the formulation of vaccines and/or for the formulation of preparations of live bacteria for oral administration to an animal or human, for instance preparations for the administration of probiotics, e.g. for the treatment of gastrointestinal disorders.

The vaccine according to the invention will be in a form suitable for oral administration, which may be a solid, semi-solid or liquid form, including but not limited to solutions and/or suspensions of the bacteria, which are usually preferred.

The vaccine preparation may also be in the form of a powder, such as a freeze dried powder that is reconstituted before use, e.g. using a suitable liquid; or in the form of a solid or liquid preparation that is (to be) mixed with solid, semi-solid or liquid food prior to administration. It may also be in the form of a fermented product.

As such, besides the bacteria, the vaccine may contain one or more pharmaceutically acceptable carriers/exipientia, such as water. The vaccine may also contain one or more adjuvants, including immune adjuvantia, suitable for oral administration, as long as these are compatible with the bacterial host and do not interfere (too much) with its desired immunogenic properties. According to one embodiment, the adjuvans may be a lactic acid bacterium, such as the bacterial host strain itself, one of the other *L. plantarum* strains mentioned above, another *Lactobacillus* species, or even a *Lactococcus, Bifidobacterium* or *Propionibacterium* species suitable for oral administration to humans or animals.

Also, the vaccine may contain one or more further therapeutic substances; and/or one or more substances that can facilitate and/or enhance the colonization of (part of) the g.i. tract by the bacteria, and/or the growth of the bacteria in the g.i. tract.

The preparation may also be in a form suitable for administration into the stomach or gut, for instance via a tube or cathether. As mentioned in the section "definitions" above, these are included within the term "oral vaccine" as used herein

Although the invention is not limited thereto, it is assumed that upon oral administration, the bacterial host settles in, and thereupon colonizes, the gastrointestinal tract, or at least a part thereof, such as the mouth, the gut, the small intestine, the large intestine, the ileum and/or the colon, and preferably the small intestine.

The antigens expressed by the bacterial host thus can come into contact with the mucosal layer, the lining and/or the wall of the g.i. tract (collectively referred to hereinbelow as *"wall of the g. i. tract"*), and more specifically with cells within said wall that can mediate an immune response against the antigen(s) thus presented, such as antigen-presenting cells (for example macrophages, dendritic cells and/or B-lymphocytes). This immunological response by the cells within the wall of the g.i. tract may by itself already constitute a significant immune response as defined herein, and/or it may trigger further immunological reactions/responses in the body of the human or animal to which the vaccine has been administered, which again may be a significant response and/or may be a protective response as defined herein.

However, the invention is not limited to any specific mechanism via which the recombinant bacterial host illicits said immune response(s). For instance, it may be that according to the invention, the immune response illicited by antigen as expressed by the bacterial host - i.e. intracellularly and/or exposed on the surface - provides a stronger/enhanced immune response compared to the response that would be illicited by the antigen as such, e.g. as a free soluble protein.

Also, according to the invention, the manner in which the antigens are presented and/or delivered to the wall of the g.i. tract and/or to specific cells therein - such as the cells which mediate and/or are involved in the immune response - may be improved/enhanced, compared to the administration, expression and/or use of the antigen as a free soluble protein. For example, as the bacterial host can adhere to the wall of the g.i. tract and can persist *in situ* for a long(er) period of time, it may be that the antigens are presented locally at the wall of the g.i. tract in a large(r) amount, at a high(er) level or concentration, in a more stable or resistant manner, and/or for a long(er) period of time, thus providing an (enhanced) immune response.

Also, it may be that the bacterial host - or any part or fragment thereof and/or any further compound(s) produced by it - interact(s) with the wall of the g.i. tract and/or with specific cells therein - such as the cells which mediate and/or are involved in the immune response - so as to enable, facilitate or enhance the immune response to the antigens associated with the bacterial host, again compared to administration, expression and/or use of the antigen as a free, soluble protein.

Also, although preferably the antigen is expressed by the baterial host such that the antigen becomes exposed on the cell surface, it is not excluded that - in order to illicit the immune reponse - the contents of the cells of the bacterial hosts are *in situ* (i.e. locally at the wall of the g.i. tract) released and/or liberated from the bacterial cell, e.g. by a mechanism which makes the walls of the bacterial cell wall permeable and/or destroys the cells or the bacterial cell wall.

Therefore, according to the invention, it is not excluded that *in situ* at the wall of the g.i. tract, the immunogenic reponse is not caused/illicited by the intact bacterial host, but by a part, fragment, fraction or compound thereof, including the antigen as such and/or cell fragments or cell fractions containing the antigen. Because of this, although the invention mainly envisages the use of vaccines containg live bacteria, which is preferred, the use of vaccines containing fragments, fractions, lysates etc., derived from the recombinant bacterial host is not excluded.

According to the invention, the amount of bacteria administered is not critical, as long as it is sufficient for the bacteria to settle into and colonize (the desired part of) the g.i. tract, and/or to cause a significant immune response. A suitable amount will be at least 10⁸ cfu, preferably 10⁸ - 10¹⁰ cfu per dose, which allows a sufficient amount of bacteria to pass the gut into the intestine, if required. Oral administration of doses less than 10⁸ cfu may not result in the desired immunogenicity (at least not in a reliable manner), whereas amounts of more than 5.10¹⁰ cfu may be cumbersome to administer orally and are therefore less preferred

The above amounts of bacteria may correspond to for instance 10⁶ to 10⁸ cfu per kg of body weight of the human or animal, although for the reasons mentioned above, the immune response obtained may not be - and usually is not - dosedependant, at least not within the range of administered amounts mentioned.

Preferably the individual to be vaccinated is a human. The human can belong to the category infant, immunocompromised person, elderly person and also can be any normally healthy infant, child or adult.

One advantage of the bacterial hosts mentioned above is that, when they are administered in the amounts indicated above, they are capable of surviving in/passing through the gut in amounts sufficient to colonize the intestine(s). Nevertheless, it is not excluded to administer the bacteria as a coated or encapsulated preparation, for instance in the form of a delayed release composition or an enteric coated composition. Suitable encapsulating compounds include but are not limited to chitosan, maltodextrin, lipids and oligo- and polysaccharides. Encapsulation may also improve the shelf-life of the vaccine.

Any of the vaccines as disclosed as such and/or in combination with another embodiment of the invention is intended to be fall within the scope of the invention. The invention covers novel recombinant strains of *Lactobacillus plantarum,* more specifically recombinant strains of *Lactobacillus plantarum 256,* said recombinant strains expressing heterologous antigen intracellularly and/or such that it is exposed on to the cell surface. Any of the embodiments of recombinant *Lactobacillus plantarum* strains described above for the novel vaccines are also covered as such by the invention. The same remark is valid for novel expression vectors suitable for high degree of intracellular expression of heterologous antigen as disclosed for the vaccine embodiments discussed above.

Furthermore, although the invention has been described hereinabove with reference to *L. plantarum* strains - which are most preferred - there may possibly be strains of other *Lactobacillus* species which may also prove suited for use as a bacterial host in providing a vaccine according to the invention, such as strains from *L. pentosus, L. reuteri, L. animalis (= L. murinus), L. fermentum, L. acidophilus, L. crispatus, L. gasseri, L. johnsonii, L. salivarius, L. brevis, L. rhamnosis* and/or *L. paracasei.*

Such strains preferably have GRAS status and more preferably are food-grade. Also, they are most preferably used in combination with the expression vector of PCT/NL95/00135 mentioned above, or another vector that gives a level of expression (e.g. intracellularly and/or exposed on the surface of the bacterial host) comparable to this preferred expression vector. As with suitable *L.plantarum* strains, it is envisaged that suitable strains can be selected by the skilled person on the basis of the description (e.g. properties and parameters) given above.

Furthermore, although the use of strains belonging to the genus *Lactobacillus* is preferred, it is envisaged that -based on the disclosure herein - suitable strains could possibly also be selected from the bifidobacteria and the propriobacteria, e.g. from the genus *Bifidobacterium* and/or the genus *Propriobacterium.* Suitable strains can be selected by the skilled person in the same way as strains from *L.*plantarum strains and/or Lactobacillus - i.e. other than from *L.plantarum -* can be selected, e.g. as mentioned above. Again, such strains preferably have GRAS status and more preferably are food-grade, and are preferably used in combination with the expression vector of PCT/NL95/00135 mentioned above, or another vector that gives a level of expression (e.g. intracellularly and/or exposed on the surface of the bacterial host) comparable to this preferred expression vector.

For instance, a suitable test for determining/confirming whether a selected strain (e.g. of *L.plantarum,* of another *Lactobacillus* species, or of a bifido- or propriobacterium) is indeed suitable as a bacterial host according to the invention, is to transform the host with the TTFC carrying vector pLP401 (for surface anchored/surface exposed expression of the TTFC-antigen) and/or the TTFC carrying vector pLP503 (for intracellular expression of the TTFC-antigen), then to administer the recombinant host thus obtained orally to an animal, preferably a mammal (e.g. a mouse such as a BALB/c and/or C57b1/6 mouse), preferably according to the single dose priming and boosting schedule as mentioned hereinbelow and shown in the Figures, followed by measuring the end-point titres of IgG in individual sera by ELISA using tetanus toxoid. All this can be carried out essentially as described in the Experimental Part, e.g. using the conditions and protocols mentioned therein.

In such an assay, the selected recombinant host preferably provides higher (i.e. at least 1% higher) titres than *L.plantarum* NICMB 8826 and/or *L.plantarum* 80 when transformed with the same vector and administered under the same conditions; and more preferably titres which are at least 10% higher, even more preferably at least 20% higher.

According to a further preferred embodiment, in such an assay, the selected recombinant host preferably provides titres which are at least 70%, more preferably at least 90% of the titres provided by *L. pluntarum* 256 transformed with the same vector and administered under the same conditions, and even more preferably titres which are at least equal to the titres provided by *L.plantarum* 256 transformed with the same vector and administered under the same conditions, although in its broadest sense, the invention is not limited thereto.

The invention will now be illustrated by means of the non-limiting Experimental part as well as the Figures mentioned below, in which:
- Fig. 1 shows expression of TTFC from *L. casei* and *L. plantarum* transformants. pLP401-TTFC transformants (surface-anchored expression) were grown in LCM (+2% mannitol) and pLP503-TTFC transformants (intracellular expression) in MRS (Difco), (both supplemented with 5µg/ml erythromycin), at 37°C to an OD 0.6, pelleted and disrupted by sonification. 30µg total protein was analysed on a 10% SDS/polyacrylamide gel and separated proteins transferred to nitrocellulose electrophoretically. TTFC was visualised with rabbit anti-TTFC (1:500) and a phosphatase/PNPP chromogen combination. Bars indicate the migration of molecular weight markers.
**(1)** 0.5ng Purified TTFC, **(2)** *L. plantarum* pLP503-TTFC, **(3)** *L.plantarum* pLP401-TTFC, **(4)** *L. plantarum* 256, **(5)** *L. casei* pLP503-TTFC, **(6)** *L. casei* 393, **(7)** MWM (kDa);
- Fig. 2. shows immunofluorescence analysis of recombinant *L. plantarum* pLP401-TTFC [ 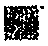 ] expressing TTFC as a surface-anchored product. Lactobacilli were gated on the basis of forward and side scatter and stained with rabbit TTFC-specific antiserum diluted 1:500 (Calbiochem, Ca). Bound antibody was detected with optimally diluted FITC-conjugated anti-rabbit IgG (Jackson, PA). Fluorescence levels from cells collected at OD 0.6 were analysed by FACScan (Becton Dickinson) and shown in histogram form, presented in relation to levels of fluorescence obtained with non-recombinant lactobacilli [■]. 10,000 cells were analysed in each experiment.
- Fig. 3A-3C show TTFC-specific IgG following immunisation of groups of 3 mice with live recombinant lactobacilli. Serum was collected from pre-immune mice and at 7 day intervals beginning on day 7. End-point titres of IgG in individual sera was measured by ELISA using microtitre plates (Nunc Maxisorp) coated o/n at 4°C with 5µg/ml of tetanus toxoid in PBS. Bound antibody was detected by the addition of anti-mouse AP conjugate (Nordic, Tilburg) and PNPP substrate. OD₄₀₅ₙₘ values of each well were measured at 90 mins. End-point titres were determined using a cut-off value calculated as the mean OD+ 2 SD's (≈0.2) of pre-immune sera diluted 1:40.

BALB/c mice were immunised intra-nasally with (**a**) 3 doses of 5x10⁹ *L. plantarum* pLP503-TTFC [●] or (**b**) 3 doses of 5x10⁹ *L. casei* pLP503-TTFC [▲] in 20µl of PBS on days 1-3. Identical booster immunisations were administered on days 28-30. C57bl/6 mice were immunised with (**c**) 5x10⁹ *L. plantarum* pLP503-TTFC [■] intra-nasally in 20µl of PBS on day 1 and day 28 and (d) 3 doses of *L. plantarum* pLP401-TTFC [ 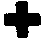 ] intra-nasally in 20µl of PBS on days 1-3 and were boosted in an identical manner on days 28-30 and 56-58 and (**e**) orally with 3 doses of 5x10⁹ *L. plantarum* pLP503-TTFC [●] in 200µl of bicarbonate buffer on days 1-3, 28-30 and 56-58.

Data points in (a) - (e) represent the geometric mean of end-point titres of groups of 3 mice ±1 standard deviation.
- Fig. 4 shows TTFC or tetanus toxoid specific proliferative responses in:
   (1) superficial cervical lymph nodes following i.n. immunisation of BALB/c mice with (**a**) 5x10⁹ *L. plantarum* pLP503-TTFC or (**b**) *L. plantarum* 256 in 20µl of PBS on day 56 or;
   (2) splenocytes following i.n. immunisation of BALB/c with (**c**) 5x10⁹ *L. plantarum* pLP503-TTFC or (**d**) *L. plantarum* 256 in 20µl of PBS on day 56.

10 days following immunisation lymphocytes were isolated and examined for [³H] thymidine incorporation following *in vitro* incubation of 4x10⁵ cells per well for 72 hours with TTFC, TT or medium alone. [³H] thymidine was added to the cultures in the last 18 hours. Results for lymph nodes are expressed as the SI calculated from the mean cpm of triplicate test cultures of cells pooled from a group of 3 mice divided by the mean cpm of cultures receiving buffer alone, and for splenocytes as the mean SI (obtained as above) for individual mice ± 1 SD.
- Fig. 5 shows the antibody respons upon boosting at different timepoints (**a**) after 4, (**b**) after 3 and (**c**) after 2 weeks, in identically primed mice (O.D. <0,2 = non-significant antibody respons). Six c57bl mice were orally immunised with 5 10⁹ L.plantarum in 200 µl bicarbonate buffer. Analysis was performed using ELISA.

### Experimental part:

The delivery of antigens to mucosal-associated lymphoid tissues in paediatric and immuno-compromised populations by safe non-invasive vectors, such as commensal lactobacilli, represents a crucial improvement to prevailing vaccination options. In this example, we describe the oral and nasal immunisation of mice with vaccines constructed through an original system for heterologous gene expression in *Lactobacillus* in which the 50kDa fragment C of tetanus toxin (TTFC) is expressed either as an intracellular or surface-anchored protein. Immunisation of mice with live recombinant lactobacilli induced significant levels of circulating TTFC-specific IgG following nasal or oral delivery of vaccine strains, substantiating their potential for safe oral delivery of paediatric vaccines.

Live microbial vaccine-vectors viable at target sites of mucosal immunisation represent efficient delivery systems to facilitate immune responses at mucosal and systemic sites concurrently. In particular, oral vaccination remains safe and inexpensive whilst maintaining the potential for single-dose immunity. Currently, vaccination against tetanus involves tetanus toxoid formulations and maintains poor coverage and contamination concerns which run concurrent with all injected vaccines. Tetanus toxin fragment C (TTFC) is the 50kDa non-toxic papain cleavage product of the tetanus holotoxin.

Commensal bacteria maintain a sophisticated non-invasive ecology with the host and although surveyed by the immune system are not necessarily susceptible to immune clearance from their ecological niches. The predominance of lactobacilli in various regions of the aero-digestive tracts indicates their particular potential as live oral vaccines. Their "generally recognised as safe" (GRAS) status is evident from applications in the food industry.

Immune homeostasis at the mucosa, in which lactobacilli participate, is a combination of physical exclusion, IgA secretion and active regulation by T-cell subsets. In this example we investigated the possibilities to avoid antigen-specific peripheral tolerance following oral delivery (oral tolerance) by vaccinating with TTFC in particulate form (contrasting soluble dietary antigens), to permit processing and presentation by mechanisms ordinarily contributing to immune regulation of intestinal flora. However, as proteolytic environments in nasal and GI tracts is a potential basis for antigen degradation we examined whether intracellular accumulation of TTFC in lactobacilli preferentially maintains epitope integrity and consequently effective immunogenicity.

*Lactobacillus spp.* appropriate as host strains were identified by quantitative cultures of faecal samples obtained following inoculation of mice with single intra-gastric doses of 10⁹ cells of a panel of rifampicin-resistent wild-type lactobacilli. *L. plantarum* 256 which persisted in the GI tract for up to 12 days and *L. casei* (ATCC 393) which became undetectable within 72 hours, were selected as prototype host-strains. Plasmid vectors were constructed for these two strains to facilitate intracellular or surface-anchored expression of TTFC. DNA coding for TTFC was adapted by PCR at its 3' or 5' end to enable cloning into the pLP401 or -501 expression plasmids.

*L. plantarum* and *L. casei* transformants containing the TTFC expression plasmids were collected in mid-exponential phase and sonicated to disrupt the bacteria with release of either cytoplasmic or cell wall-bound proteins. Proteins were separated by SDS-PAGE and immunoblots developed using TTFC-specific rabbit antiserum. As shown in Fig. 1, bacteria containing vector pLP401-TTFC express a surface-anchored 75kDa polypeptide and bacteria containing pLP503-TTFC express an intracellular 50kDa polypeptide. Expression of TTFC on the cell-wall of *L*. *plantarum* was confirmed by FACscan (Fig. 2).

Intra-nasal (i.n.) immunisation of BALB/c or C57b1/6 mice was undertaken on days 1 or 1-3 with 5x10⁹ lactobacilli and booster immunisations administered identically on days 28 or 28-30. For oral immunisation identical doses of recombinant lactobacilli were administered oro-gastrically in a 200µl volume of 0.2M NaHCO₃, following a similar schedule. Serum IgG levels were evaluated as previously described using microtiter plates (Maxisorp, Nunc) coated o/n with 50µl of a 5µg/ml solution of tetanus toxoid (RIVM, Bilthoven, The Netherlands) in PBS (Robinson et al., Nature Biotechnology. 1997. **15**:653-657). Lymphocyte proliferation was measured as previously described using [³H] thymidine incorporation (Obeid et al., 1993. J. Gen. Virol).

BALB/c mice receiving three i.n. doses of *L. plantarum* or *L. casei* expressing intracellular TTFC on days 1-3 were strongly primed for a secondary response to TTFC following booster i.n. administrations on days 28-30. The titre of the TTFC-specific response to recombinant *L. plantarum* (Fig 3a) was higher than to recombinant *L. casei* (Fig. 3b) with IgG detectable as rapidly as day 21, rising to titres of 10^{3.6} and 10^{2.9} by day 49, respectively. Furthermore, cervical lymph node cells in addition to splenocytes, isolated from BALB/c mice immunised i.n. with the recombinant *L. plantarum* strains proliferated *in vitro* when restimulated with either TTFC or tetanus toxoid (Fig. 4a+c), suggesting that i.n. immunisation induced specific immunity via NALT activation. No antigen-specific proliferation was observed in cells obtained from mice immunised identically with wildtype *L. plantarum* (Fig. 4b+d).

In C57b1/6 immunised i.n. with the more immunogenic *L. plantarum* transformant that expressed TTFC intracellularly using a single dose priming and booster schedule, (Fig. 3c) higher mean titres of serum IgG (10^{4.2}) were induced by day 35 rising to 10^{4.5} by day 49.

In contrast, C57bl/6 mice immunised intranasally with *L. plantarum* expressing TTFC at the cell surface, required the three dose priming and booster schedule to induce TTFC-specific (Fig. 3d). *L. casei* expressing TTFC on the cell surface, however, failed to demonstrate immunogenicity in either BALB/c or C57bl/6 strains as did the *L. plantarum* transformant administered on days, 1, 28 and 56 as single doses (data not shown).

This example provides a significant endorsement for *Lactobacillus*-based vaccines by extending the observations of immunogenicity following nasal immunisation to demonstrate that oral immunisation of C57bl/6 mice with 5x10⁹ *L. plantarum* expressing TTFC intracellularly induces TTFC-specific serum IgG responses (Fig. 3e) following a second booster administered on days 63-65 and which increased to a titre of 10³ by day 77. BALB/c or C57bl/6 mice immunised orally with the *L.casei* transformants (expressing TTFC either intracellularly or surface-anchored) and the *L plantarum* expressing TTFC on the cell surface failed to induce detectable TTFC-specific serum IgG responses at all equivalent time points examined (data not shown). However, the results obtained in C57bl/6 with *L. plantarum* expressing the intracellular TTFC represents the first report of both efficient expression of TTFC in non-pathogenic *Lactobacillus* transformants and induction of immunity to TTFC following oral immunisation. Mice receiving wild-type lactobacilli or irrelevant vectors deomonstrated no TTFC-specific serum IgG responses at any time point (data not shown).

The pLP401- /501- plasmids uniquely comprise of fully homologous *Lactobacillus* gene expression elements, enabling directed and regulatable expression of TTFC at levels and efficiencies not previously attainable in *Lactobacillus.* Importantly, FACScan confirms transport of the TTFC to the cell-wall but indicates that surface exposition of TTFC does not occur in *L. plantarum* at an identical efficiency to that previously reported for *L. casei.* This might reflect differences in the structure of the cell wall and translocation system inherent to the host strains. We found that the majority of recombinant *L. casei* pLP401-TTFC express TTFC on the cell surface. However, surface antigen expression may be particularly susceptible to low pH, bile-acid or proteolytic environments encountered by vaccine-vectors following mucosal immunisation. In this example we show that the *L. plantarum* pLP401-TTFC recombinant strains, although expressing relatively low levels of surface-exposed TTFC, demonstrate an immunogenicity following i.n. administration which is undetectable with *L. casei* pLP401-TTFC. Therefore high levels of "stable" surface expression may not be as critical as intracellular TTFC levels for ensuring sufficient antigen remains available to immune-inductive sites and the variations in both the levels of TTFC expression and persistence in the GI-tract between the recombinant strains may have pivotal influences on the potency of the recombinant vaccine. Nevertheless, even the *L. plantarum* transformants expressing TTFC at the bacterial surface fail to induce TTFC-specific antibodies following only a single shot i.n. immunisation schedule suggesting that high levels of "stable" surface expression are critical under these conditions. The results from immunoblot analyses (Fig 1) indeed show that relatively low TTFC quantities are expressed by the pLP401-TTFC transformants, in comparison to those observed with *L. plantarum* pLP503-TTFC. However, the efficiency of the nasal route for immunisation is comprehensively documented and nasal epithelium possesses specialised M-like cells also found overlying Peyer's patches in the GI-tract (Wu et al., Infect. Immun. 1997 65:227-235).

The GI-tract in contrast is not an efficient site for induction of immune responses and peripheral tolerance induction following oral administration of antigens must always be a consideration in vaccination strategies such as these, particularly when the carrier organism persists (albeit transiently) in the intestines. We have demonstrated that appropriate host *Lactobacillus* strains enable TTFC expression at levels suitable for immunogenicity following oral immunisation. Peripheral tolerance induction following the oral administration of recombinant *L. plantarum* has, perhaps surprisingly, not occurred and these results open up the possibility of exploiting as host strains "probiotic" bacteria which currently represent routine constituents of a variety of foods such as yoghurt. A more defined analysis of host-vector combinations may provide options for accomplishing single-dose immunity equivalent to that observed following nasal delivery by sustaining the exposure of TTFC to the M-cells and Peyer's patches of the GI tract by lactobacilli which adhere or persist. Intrinsic levels of antigen expression may be equally but these antigen quantities may be rapidly cleared from the murine GI-tract through proteolytic degradation or rapid peristaltic clearance. However, plasmid retention in *L. casei* and *L. plantarum* strains re-isolated from the faeces following immunisation is very high (data not shown) indicating that *in vivo* expression of the TTFC could potentially occur. Furthermore, the less hostile environment of the nasal tract may make restraints associated with antigen clearence or degradation less apparent, as shown in Fig. 3a-d.

This first demonstration of TTFC immunogenicity following delivery of recombinant lactobacilli by the oral route has at last endorsed the continued development of a safe *Lactobacillus*-based oral neonatal tetanus vaccine to combat the current 400,000 deaths recorded annually. Accelarating the kinetics and increasing serum IgG titres of the response, by defining optimal host-vector combinations, will provide a sound basis to analyse protective efficacy against lethal toxin challenge as well as an expanded application of this technology to a catalogue of additional pathogens which manifest their pathology through the mucosal surfaces.

## Claims

1. An oral vaccine comprising recombinant lactic acid bacteria expressing heterologous antigen in vivo intracellularly and/or the surface of the lactic acid bacterium as specific immunogenicity eliciting component for eliciting immunogenicity against the heterologous antigen, characterised in that the recombinant lactic acid bacterium is a *Lactobacillus plantarum.*

2. A vaccine according to claim 1, wherein the recombinant *Lactobacillus plantarum* comprises an expression vector capable of expressing the heterologous antigen intracellularly and/or such that the heterologous antigen is exposed on the cell surface under conditions present in the gastrointestinal tract.

3. A vaccine according to claim 1 or 2, wherein the heterologous antigen is specific for inducing immunogenicity against a pathogenic microorganism, suitably a heterologous antigen specific for mucosa colonising pathogens or pathogens entering the body via the mucosa, specifically via the oral route.

4. A vaccine according to any of the preceding claims, wherein the heterologous antigen is specific for inducing immunogenicity against a pathogenic microorganism colonising the gastrointestinal tract.

5. A vaccine according to any of the preceding claims, wherein the pathogenic microorganism is selected from the group consisting of herpes virus, rubella virus, influenza virus, mumps virus, measles virus, poliomyelitis virus, rotavirus, respiratory syncytial virus, *Campylobacter* species, *Chlamydial* organisms, species of the genus *Cryptosporidium,* cytomegalovirus, human immounodeficiency virus, *Actinomyces* species, *Entamoeba histolytica,* arenaviruses, arboviruses, *Clostridium botulinum,* species of the genus *Candida, Vibrio cholera, Cryptococcus neoformans,* EHEC strains of *E.coli* O157:H7, O26:H11, O111:H8 and O104:H21, ETEC strains of *E. coli,* strains of *E.coli* shown to possess enteroinvaisiveness (EIEC), EPEC strains of *E.coli,* EAggEC strains of *E.coli.,* DAEC strains of *E.coli,* filoviridae, parvovirus, *Filarioidea, Staphylococcus aureus,* species of the genus *Clostridium perfringens, Helicobacter pylori,* Caliciviruses, *Giacardia lamblia, Neisseria gonorrhoeae,* hantaviruses, hepatitis viruses types A, B, C, D, E, *Legionellae* strains, *Mycobacterium leprae, Listeria monocytogenes,* species of the genus *Clostridium perfringens, Borrelia burgdorferi, Pseudomonas pseudomallei,* Epstein Barr virus, *Onchocerca volvulus,* Poxviruses, *Bordetella pertussis, Yersinia pestis, Coxiella burnetti,* rabies virus, *Treponema pallidium, Mycobacterium tuberculosis, Salmonella typhi,* eukaryotic parasite causing malaria, *pneumocystis pneumonia,* as well as agents causing toxoplasmosis, or any combination thereof, and preferably chosen from rotavirus, respiratory synctial virus, Mycobacterium tuberculosis, human immunodeficeincy virus, E.coli, Vibrio cholera, streptococci and chlamydia, or any combination thereof.

6. A vaccine according to any of the preceding claims, wherein the heterologous antigen is selected from the group consisting of viral and/or bacterial antigens such as the gp160 envelope protein of the HIV virus, the surface glycoprotein of a *Leishmania* parasite, Shiga-like toxin, *Shigella* lipopolysaccharide antigen, *Escherichia coli* fimbrial antigen, a CFA antigen of an enterotoxigenic *Escherichia coli* strain, anthrax toxin, pertussis toxin, tetanus toxin.

7. A vaccine according to claims 1-4, wherein the heterologous antigen is a human allergen.

8. A vaccine according to any of the preceeding claims wherein the heterologous antigen is specific for tetanus.

9. A vaccine according to any of the preceding claims wherein the recombinant *Lactobacillus plantarum* comprises an expression vector capable of expressing the heterologous antigen intracellularly and/or such that the heterologous antigen is exposed on the cell surface to a degree sufficient to induce protective immunogenicity.

10. A vaccine according to any of the preceding claims formulated as a single dose vaccine.

11. A vaccine according to any of the preceding claims, wherein the recombinant Lactobacillus plantarum comprises an expression vector capable of expressing the heterologous antigen intracellularly and/or exposing the heterologous antigen to the cell surface to a degree exceeding that of the vector disclosed for *Lactobacillus plantarum* 80 beta galactosidase expression.

12. A vaccine according to any of the preceding claims, wherein the recombinant Lactobacillus plantarum comprises homologous expression and/or secretion signals on the expression vectors for *Lactobacilli,* preferably for *Lactobacillus plantarum.*

13. A vaccine according to any of the preceeding claims, wherein the recombinant *Lactobacillus plantarum* strain exhibits a persistance in the individual to be immunised exceeding 5 days, preferably exceeding 9 days, suitably more than 15 or even 20 days

14. A vaccine according to any of the preceeding claims wherein the recombinant *Lactobacillus plantarum* exhibits a persistance longer than that of L plantarum 80 and preferably longer than that of L plantarum NCIMB 8826 under equivalent conditions.

15. A vaccine according to any of the preceeding claims suitably formulated to be administered safely to a human, e.g. belonging to the category infant, immunocompromised person, elderly person and also any normally healthy infant, child or adult.

16. A vaccine according to any of the preceeding claims, wherein the recombinant *Lactobacillus planturum* is a recombinant *Lactobacillus plantarum* 256.

17. A vaccine according to any of the preceeding claims wherein the vaccine is free of adjuvant.

18. A vaccine according to any of the preceeding claims wherein the vaccine comprises at least one further additive useful in the field of immunisation, e.g. an adjuvant, a pharmacologically acceptable carrier.

19. A recombinant *Lactobacillus plantarum,* more specifically a recombinant strain of *Lactobacillus plantarum* 256 as defined in any of the preceeding vaccine claims.

20. An expression vector suitable for intracellular expression or exposure of a heterologous antigen encoded thereon, said expression vector providing expression in a *Lactobacillus plantarum* of the heterologous antigen under conditions existing in the gastrointestinal tract.
